# EUROPEAN PATENT SPECIFICATION

(11) **EP 4 513 433 B1**
(45) Date of publication and mention of the grant of the patent: **23.09.2026**
(21) Application number: 24182201.4
(22) Date of filing: 14.06.2024
(51) Int. Cl.: G06T 7/11, G16H 30/40, G16H 50/20, G16H 50/70

(54) **LESION DETECTION METHOD AND LESION DETECTION PROGRAM**
LÄSIONSDETEKTIONSVERFAHREN UND LÄSIONSDETEKTIONSPROGRAMM
PROCÉDÉ ET PROGRAMME DE DÉTECTION DE LÉSION

(30) Priority: 05.07.2023 JP 2023110444
(43) Date of publication of application: 26.02.2025
(73) Proprietor: FUJITSU LIMITED, Kawasaki-shi, Kanagawa 211-8588 (JP)
(72) Inventor: MIYAZAKI, Nobuhiro, Kawasaki-shi, Kanagawa, 211-8588 (JP); TAKEBE, Hiroaki, Kawasaki-shi, Kanagawa, 211-8588 (JP); BABA, Kozo, Kawasaki-shi, Kanagawa, 211-8588 (JP); BABA, Takayuki, Kawasaki-shi, Kanagawa, 211-8588 (JP)
(74) Representative: Hoffmann Eitle

(56) References cited:
- JP-A- 2022 155 871
- US-A1- 2021 401 392
- US-A1- 2023 081 693
- BRIA ALESSANDRO ET AL: "Addressing class imbalance in deep learning for small lesion detection on medical images", vol. 120, 1 May 2020 (2020-05-01), US, pages 103735, XP093209813, ISSN: 0010-4825, Retrieved from the Internet <URL:https://pdf.sciencedirectassets.com/271150/1-s2.0-S0010482520X00047/1-s2.0-S0010482520301177/main.pdf?X-Amz-Security-Token=IQoJb3JpZ2luX2VjEOr//////////wEaCXVzLWVhc3QtMSJGMEQCIAhJjUJBfVRXkxYkDnThu17iYA4EcUT48Ox6CGsNTfPmAiB7Sq8xT0Omzz6I0AKUeOR0+dkr3lQ4eyjLMP6615ciuCqzBQgzEAUaDDA1OTAwMzU0Njg2NSIMfewnl> DOI: 10.1016/j.compbiomed.2020.103735

## Description

### FIELD

Embodiments discussed herein are related to a lesion detection method and a lesion detection program.

### BACKGROUND

Medical images obtained by computed tomography (CT), magnetic resonance imaging (MRI), or the like are widely used for diagnosis of various diseases. For image diagnosis using the medical images, a doctor has to interpret a large number of images, which imposes a large burden on the doctor. For this reason, there is a demand for a technique for supporting diagnosis work of the doctor by a computer in some form.

As an example of such a technique, there is a technique for detecting a lesion region from a medical image by using a trained model (machine learning model) generated by machine learning. For example, a system for heart segmentation that trains a neural network integrated with a long-short-term memory (LSTM) is proposed.

US 2021/0401392 A1 relates to techniques for segmenting tumors with positron emission tomography (PET) using deep convolutional neural networks for image and lesion metabolism analysis. Particularly, aspects of the present disclosure are directed to obtaining a PET scans and computerized tomography (CT) or magnetic resonance imaging (MRI) scans for a subject, preprocessing the PET scans and the CT or MRI scans to generate standardized images, generating two-dimensional segmentation masks, using two-dimensional segmentation models implemented as part of a convolutional neural network architecture that takes as input the standardized images, generating three-dimensional segmentation masks, using three-dimensional segmentation models implemented as part of the convolutional neural network architecture that takes as input patches of image data associated with segments from the two-dimensional segmentation mask, and generating a final imaged mask by combining information from the two-dimensional segmentation masks and the three-dimensional segmentation masks.

US 2023/0081693 A1 relates to a learning device includes at least one processor. The processor acquires a medical image to be detected, acquires apparatus identification information for identifying an imaging apparatus that has captured the medical image to be detected, selects any one of a plurality of lesion detection models, which detect a lesion from the medical image, on the basis of the apparatus identification information, and detects the lesion from the medical image to be detected, using the selected lesion detection model.

U.S. Patent Application Publication No. 2019/0223725 is disclosed as related art.

### PROBLEMS

As an example of the machine learning model, there is a machine learning model that classifies whether or not each pixel included in a captured tomographic image is a specific lesion region. In the classification processing using the machine learning model, when one tomographic image is input to the machine learning model, a lesion region is classified in pixel units from the tomographic image.

A problem with such a machine learning model is that classification accuracy may be decreased depending on an imaging position of the tomographic image in a human body.

For example, when such a machine learning model is generated, the actually captured tomographic image of the human body is used as learning data. Normally, since the tomographic image is captured at equal intervals from a specific range inside any human body, the number of tomographic images used as the learning data is fixed regardless of the position in the human body. On the other hand, an appearance frequency of a lesion region may have a bias depending on the position inside the human body. Therefore, among the tomographic images used as the learning data, the number of tomographic images including the lesion region may vary depending on the position inside the human body. For a tomographic image captured at a position at which a sufficient number of tomographic images are not used as the learning data, there is a possibility that the classification accuracy of the lesion is decreased.

According to one aspect, an object of the present disclosure is to provide a lesion detection method and a lesion detection program capable of detecting a lesion region with a certain level of accuracy regardless of an imaging position of a tomographic image of an inside of a human body.

This object is accomplished by the subject-matter of the independent claims. The dependent claims concern particular embodiments.

### [Effects of Invention]

According to one aspect, a lesion region may be detected with a certain level of accuracy regardless of an imaging position of a tomographic image of an inside of a human body.

### BRIEF DESCRIPTION OF DRAWINGS

FIG. 1 is a diagram illustrating a configuration example and a process example of a lesion detection apparatus according to a first embodiment;
FIG. 2 is a diagram illustrating a configuration example of a diagnosis support system according to a second embodiment;
FIG. 3 is a diagram illustrating a first example of a trained model for lesion classification;
FIG. 4 is a diagram illustrating a second example of the trained model for lesion classification;
FIG. 5 is a diagram illustrating a bias in the number of pieces of learning data in an organ region;
FIG. 6 is a diagram illustrating an example of selecting a lesion classification model;
FIG. 7 is a diagram illustrating a specific example of model selection in accordance with the number of pieces of learning data of a lesion region;
FIG. 8 is a diagram illustrating a configuration example of a processing function provided by a diagnosis support apparatus;
FIG. 9 is a diagram illustrating an example of data stored in a learning data storage unit;
FIG. 10 is a diagram illustrating an example of data stored in a setting data storage unit;
FIG. 11 is a diagram illustrating an example of data stored in an aggregation data storage unit;
FIG. 12 is a diagram illustrating an example of data stored in a model data storage unit;
FIG. 13 is an example of a flowchart illustrating a procedure of a data aggregation process;
FIG. 14 is an example of a flowchart illustrating a procedure of a model determination process;
FIG. 15 is an example of a flowchart illustrating a procedure of a model generation process;
FIG. 16 is an example of a flowchart illustrating a procedure of a lesion classification process;
FIG. 17 is a diagram illustrating a configuration example of a processing function provided by a diagnosis support apparatus according to a third embodiment;
FIG. 18 is a diagram illustrating an example of data stored in the aggregation data storage unit;
FIG. 19 is another example of the flowchart illustrating the procedure of the data aggregation process; and
FIG. 20 is an example of a flowchart illustrating a procedure of a model determination process.

### DESCRIPTION OF EMBODIMENTS

Hereinafter, embodiments of the present disclosure will be described with reference to the drawings.

### [First Embodiment]

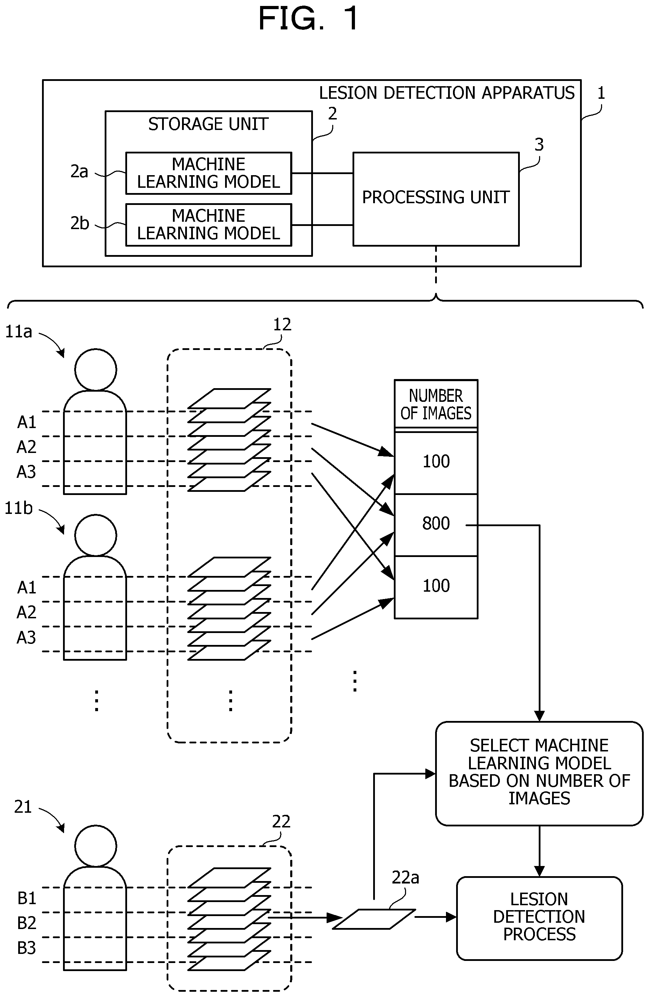

FIG. 1 is a diagram illustrating a configuration example and a process example of a lesion detection apparatus according to a first embodiment. A lesion detection apparatus 1 illustrated in FIG. 1 is an information processing apparatus that detects a specific lesion region from a tomographic image obtained by imaging an inside of a human body. As an example in the following description, it is assumed that the tomographic image is an image captured at certain intervals in a height direction of the human body. The lesion detection apparatus 1 includes, for example, a storage unit 2 and a processing unit 3.

The storage unit 2 is a storage region secured in a storage device (not illustrated) included in the lesion detection apparatus 1. The storage unit 2 stores data indicating a machine learning model 2a and data indicating a machine learning model 2b.

The machine learning model 2a is a trained model that classifies whether or not a pixel included in an input tomographic image is a specific lesion region in units of the pixel. In the classification processing using the machine learning model 2a, when one tomographic image is input to the machine learning model 2a, a lesion region is classified in pixel units from the tomographic image.

On the other hand, the machine learning model 2b is a trained model that classifies whether or not an image block obtained by dividing the input tomographic image into certain sizes is a specific lesion region in units of the image block. When one tomographic image is given in the classification processing using the machine learning model 2b, the tomographic image is divided into image blocks. The image blocks are input to the machine learning model 2b one by one, and it is determined whether or not the input image block is a lesion region each time.

The processing unit 3 is, for example, a processor. In this case, the following process of the processing unit 3 is implemented by the processor executing a predetermined program. By selectively using any one of the machine learning models 2a and 2b, the processing unit 3 detects a lesion region from the captured tomographic image.

A tomographic image group 12 is a set of tomographic images including a lesion region, among tomographic images used as learning data when the machine learning model 2a is generated by machine learning. A tomographic image included in the tomographic image group 12 is an image obtained by imaging an inside of human bodies 11a, 11b, and ....

The processing unit 3 acquires a tomographic image 22a obtained by imaging a first imaging position of an inside a human body 21 as a target for lesion detection. Among the tomographic images included in the tomographic image group 12, the processing unit 3 selects any one of the machine learning models 2a and 2b, based on the number of tomographic images captured at a second imaging position corresponding to the first imaging position in the human bodies 11a, 11b, and .... By using the selected machine learning model, the processing unit 3 detects a specific lesion region from the tomographic image 22a.

An appearance frequency of the lesion region may have a bias depending on a position of the inside of the human body. Therefore, among the tomographic images used as learning data, the number of tomographic images including the lesion region may vary depending on the position of the inside of the human body. For a tomographic image captured at a position at which a sufficient number of tomographic images are not used as learning data, there is a possibility that classification accuracy of a lesion using the machine learning model 2a is decreased.

By the model selection described above, the processing unit 3 selects the machine learning model 2a in a case where it is determined that the number of tomographic images captured at the second imaging position is a sufficient number with which a certain or higher level of lesion classification accuracy is obtained from the machine learning model 2a. On the other hand, in a case where it is determined that the number of images is not sufficient to obtain the certain or higher level of lesion classification accuracy from the machine learning model 2a, the processing unit 3 selects the machine learning model 2b. Since the machine learning model 2b clasifies a lesion region based on a feature in a division region instead of the entire tomographic image, the lesion classification accuracy does not depend on the imaging position of the tomographic image. Therefore, by the model selection described above, it is possible to detect a lesion region with a certain level of accuracy regardless of the imaging position of the tomographic image of the inside of the human body.

According to the example illustrated in FIG. 1, the lesion detection apparatus 1 or another information processing apparatus counts the number of tomographic images included in the tomographic image group 12 for each position in the human bodies 11a, 11b, and .... Specifically, a predetermined region of the human bodies 11a, 11b, and ... is divided into three regions at equal intervals in a direction perpendicular to the tomographic image, so that division regions A1 to A3 are set. For each of the division regions A1 to A3, the number of tomographic images is counted. In the example illustrated in FIG. 1, it is assumed that the tomographic image group 12 includes 1000 tomographic images, the number of images in the division region A1 is 100, the number of images in the division region A2 is 800, and the number of images in the division region A3 is 100.

Noted that the division regions A1 to A3 are obtained by dividing a region between predetermined reference points in each of the human bodies 11a, 11b, and .... As such a region, a specific organ region may be applied.

In the example illustrated in FIG. 1, a tomographic image group 22 is acquired by imaging a predetermined region inside the human body 21. By dividing the predetermined region of the human body 21 into three regions at equal intervals in a direction perpendicular to the tomographic image 22a, division regions B1 to B3 are generated. It is assumed that among tomographic images included in the tomographic image group 22, a lesion detection process is executed on the tomographic image 22a belonging to the division region B2. In this case, the processing unit 3 acquires the number of tomographic images used as learning data, which is counted for the division region A2 corresponding to the division region B2, among the division regions A1 to A3. Here, the number of images "800" is acquired for the division region A2.

As an example, it is assumed that 20% of a total number (1000 in FIG. 1) of the tomographic images included in the tomographic image group 12 is set as a threshold value. Since a ratio of the acquired number "800" of images to the total number "1000" is 80%, which is more than the threshold value, the processing unit 3 selects the machine learning model 2a. In this case, the processing unit 3 inputs the tomographic image 22a to the machine learning model 2a, and classifies whether or not each pixel of the tomographic image 22a is a lesion region.

On the other hand, for example, it is assumed that among the tomographic images included in the tomographic image group 22, the lesion detection process is executed on a tomographic image belonging to the division region B3. In this case, the processing unit 3 acquires the number of tomographic images used as learning data, which is counted for the division region A3 corresponding to the division region B3, among the division regions A1 to A3. Here, the number of images "100" is acquired for the division region A3. Since a ratio of the acquired number "100" of images to the total number "1000" is 10%, which is equal to or less than the threshold value, the processing unit 3 selects the machine learning model 2b. In this case, the processing unit 3 divides the tomographic image into image blocks, and inputs the divided image blocks one by one to the machine learning model 2b to classify whether each image block is a lesion region.

With such a process, it is possible to detect a lesion region with a certain level of accuracy regardless of an imaging position of a tomographic image of an inside of a human body.

### [Second Embodiment]

Next, a system capable of detecting a lesion region of a lung from a CT image will be described.

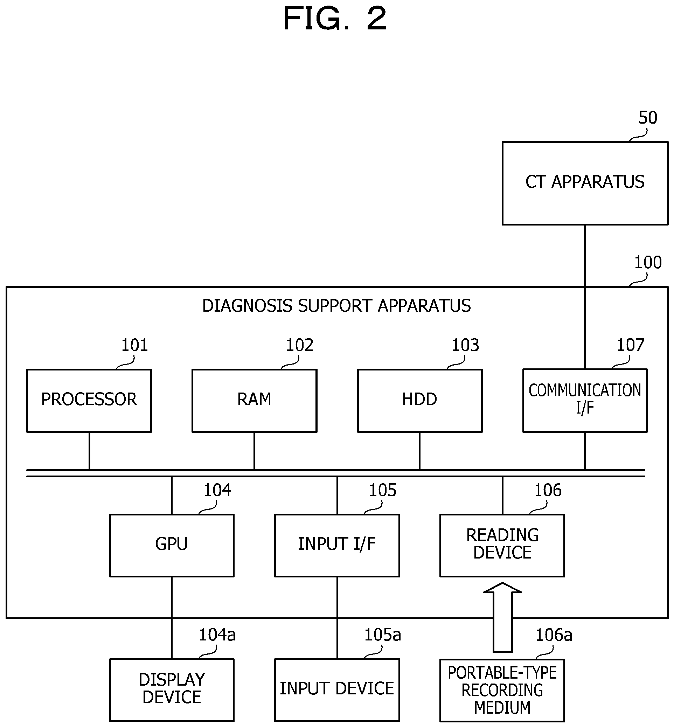

FIG. 2 is a diagram illustrating a configuration example of a diagnosis support system according to a second embodiment. The diagnosis support system illustrated in FIG. 2 is a system that supports image diagnosis work by CT imaging, and includes a CT apparatus 50 and a diagnosis support apparatus 100. Noted that the diagnosis support apparatus 100 is an example of the lesion detection apparatus 1 illustrated in FIG. 1.

The CT apparatus 50 captures an X-ray CT image of a human body. According to the present embodiment, the CT apparatus 50 captures a predetermined number of tomographic images of an axial plane in a chest region while changing a position (slice position) of a human body in a height direction (direction perpendicular to the axial plane) at a predetermined interval.

The diagnosis support apparatus 100 extracts a lung field region from each tomographic image captured by the CT apparatus 50, and detects a lesion region based on image information of the extracted lung field region. According to the present embodiment, a shadow region is detected as the lesion region. The diagnosis support apparatus 100 may classify a type of shadow. For example, the diagnosis support apparatus 100 causes a display device to display information indicating a detection result of the lesion region. Thus, the diagnosis support apparatus 100 supports the image diagnosis work of a user (for example, a radiologist).

The diagnosis support apparatus 100 detects a lesion region (or classifies a lesion) by using a trained model generated in advance by machine learning. The diagnosis support apparatus 100 is also capable of executing a process of generating the trained model by machine learning.

A hardware configuration of the diagnosis support apparatus 100 will be described below with reference to FIG. 2. The diagnosis support apparatus 100 is implemented as a computer illustrated in FIG. 2, for example. As illustrated in FIG. 2, the diagnosis support apparatus 100 includes a processor 101, a random-access memory (RAM) 102, a hard disk drive (HDD) 103, a graphics processing unit (GPU) 104, an input interface (I/F) 105, a reading device 106, and a communication interface (I/F) 107.

The processor 101 comprehensively controls the entire diagnosis support apparatus 100. The processor 101 is, for example, a central processing unit (CPU), a microprocessor unit (MPU), a digital signal processor (DSP), an application-specific integrated circuit (ASIC), or a programmable logic device (PLD). The processor 101 may also be a combination of two or more elements among the CPU, the MPU, the DSP, the ASIC, and the PLD. Noted that the processor 101 is an example of the processing unit 3 illustrated in FIG. 1.

The RAM 102 is used as a main storage device of the diagnosis support apparatus 100. The RAM 102 temporarily stores at least a part of an operating system (OS) program and an application program to be executed by the processor 101. The RAM 102 also stores various kinds of data to be used in processes performed by the processor 101.

The HDD 103 is used as an auxiliary storage device of the diagnosis support apparatus 100. The OS program, the application program, and various kinds of data are stored in the HDD 103. Noted that a different type of nonvolatile storage device such as a solid-state drive (SSD) may be used as the auxiliary storage device.

A display device 104a is coupled to the GPU 104. The GPU 104 causes the display device 104a to display an image in accordance with an instruction from the processor 101. Examples of the display device 104a include a liquid crystal display, an organic electroluminescence (EL) display, and the like.

An input device 105a is coupled to the input interface 105. The input interface 105 transmits a signal output from the input device 105a to the processor 101. The input device 105a includes a keyboard, a pointing device, and the like. The pointing device includes a mouse, a touch panel, a tablet, a touch pad, a track ball, and the like.

A portable-type recording medium 106a is removably attached to the reading device 106. The reading device 106 reads data recorded in the portable-type recording medium 106a, and transmits the data to the processor 101. The portable-type recording medium 106a includes an optical disc, a semiconductor memory, and the like.

The communication interface 107 transmits and receives data to and from another apparatus such as the CT apparatus 50 via a network.

The hardware configuration as described above is capable of implementing a processing function of the diagnosis support apparatus 100.

As described above, a lesion classification process from a medical image may be executed by using a trained model generated by machine learning, for example.

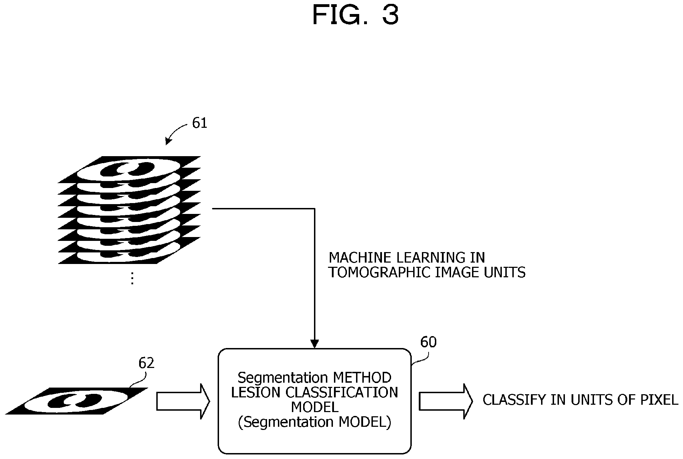

FIG. 3 is a diagram illustrating a first example of a trained model for lesion classification. A lesion classification model 60 illustrated in FIG. 3 is a trained model for classifying a lesion by a Segmentation method. Hereinafter, the lesion classification model 60 may be referred to as a "Segmentation model 60". Upon receiving an input of a tomographic image 62, the Segmentation model 60 classifies whether or not a pixel of the tomographic image 62 is a specific lesion region (whether or not the pixel is a shadow in the present embodiment) in units of the pixel. Here, it is assumed that the tomographic image 62 is a tomographic image of an axial plane.

In the same manner, the Segmentation model 60 is generated by machine learning (for example, deep learning) using a tomographic image group 61 of the axial plane as learning data. A label indicating whether or not each pixel is a lesion region is added to each tomographic image used as learning data, and these labels are used as correct answer data in machine learning. By performing machine learning in tomographic image units by using such learning data, the Segmentation model 60 is generated.

Noted that for each pixel, the Segmentation model 60 may be a lesion classification model that classifies three or more types of lesions including a type indicating that the pixel is not a lesion. For example, the Segmentation model 60 classifies, for each pixel, one of six types of shadows of a ground glass opacity, a consolidation, a honeycomb, emphysema, other shadows, and a normal (not a shadow). In this case, a label indicating which of the shadow types described above is added to each tomographic image used as the learning data for each pixel. The lesion classification process using such a Segmentation model 60 is effective for, for example, diagnosis of a diffuse pneumonia.

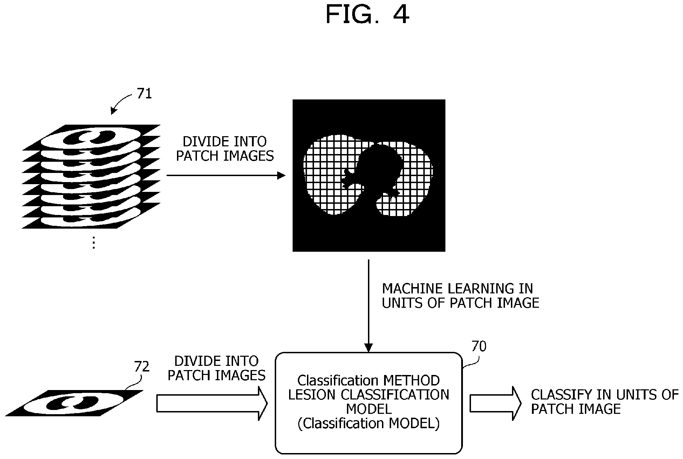

FIG. 4 illustrates a second example of the trained model for lesion classification. A lesion classification model 70 illustrated in FIG. 4 is a trained model for classifying a lesion by a Classification method. Hereinafter, the lesion classification model 70 may be referred to as a "Classification model 70". Upon receiving an input of a tomographic image 72, the Classification model 70 divides the tomographic image 72 into patch images (image blocks) having a certain size, and classifies whether or not the divided patch image is a specific lesion region (whether or not the divided patch image is a shadow in the present embodiment) in units of the divided patch image. Noted that in the same manner as the tomographic image 62 in FIG. 3, it is assumed that the tomographic image 72 is a tomographic image of an axial plane.

The classification model 70 is generated by machine learning (for example, deep learning) using a tomographic image group 71 of an axial plane as learning data. Meanwhile, unlike the Segmentation method in FIG. 3, each tomographic image used as learning data is divided into the patch images described above, and a label indicating whether or not the patch image is a lesion region is added to each of the divided patch images. The Classification model 70 is generated by performing machine learning in which the patch image is used as a unit, by using each patch image as teaching data and using the label as correct answer data.

Noted that for each patch image, the Classification model 70 may be a lesion classification model that classifies three or more types of lesions including a type indicating that the patch image is not a lesion. For example, the Classification model 70 classifies, for each patch image, one of the six types of shadows described above. In this case, a label indicating one of the shadow types described above is added to each tomographic image used as the learning data for each patch image.

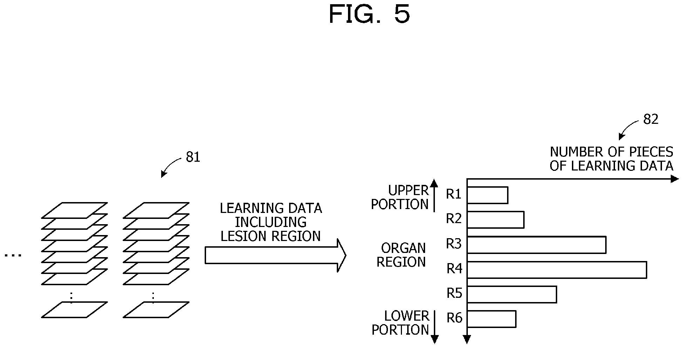

In this manner, the Classification model 70 classifies a lesion by using a patch image including a plurality of pixels as a unit. On the other hand, since the Segmentation model 60 classifies a lesion in pixel units, it is possible to classify the lesion by using a finer region in a tomographic image as a unit, as compared with the Classification model 70. Meanwhile, as illustrated in FIG. 5 described below, the Segmentation model 60 has a problem in that there may be a difference in classification accuracy for each position of tomographic images in an organ region.

FIG. 5 is a diagram illustrating a bias in the number of pieces of learning data in an organ region. In generation of the Segmentation model 60 by machine learning, a tomographic image of an organ region (lung field region in the present embodiment) among tomographic images obtained by imaging a chest region of a human body is used as learning data. A learning data set 81 illustrated in FIG. 5 is a set of pieces of learning data including, among such learning data, a tomographic image including a lesion region (a tomographic image including one or more pixels of the lesion region) as teaching data. For example, the learning data set 81 does not include a tomographic image having no lesion region. Hereinafter, the learning data included in the learning data set 81 may be referred to as "learning data of lesion region".

A histogram 82 illustrated in FIG. 5 is a graph illustrating the number of pieces of learning data included in the learning data set 81 for each of division regions obtained by equally dividing the organ region in a vertical direction. As an example, the histogram 82 in FIG. 5 indicates the number of pieces of learning data for each of division regions R1 to R6 obtained by dividing the organ region into six regions from an upper side.

Where a lesion region is likely to appear in the organ region differs for each lesion as an classification target. In a case where an appearance frequency of the lesion region has a bias depending on a position in the organ region, the number of pieces of learning data of the lesion region included in the learning data set 81 described above also has a bias depending on the position of the organ region. In the example illustrated in FIG. 5, the lesion region is likely to appear around a center of the organ region in the vertical direction. Therefore, in the histogram 82, the number of pieces of learning data around the center in the vertical direction is larger than the number of pieces of learning data at the upper portion or the lower portion.

Based on an image feature in the entire one tomographic image such as a shape of an organ in the tomographic image or a positional relationship with another organ, the Segmentation model 60 classifies a lesion in pixel units. In order to classify the lesion with high accuracy, the sufficient number of pieces of learning data as learning data of the lesion region are desired for each position in the organ region. Meanwhile, in a case where there is a bias in the number of pieces of learning data for each position in the organ region as in the histogram 82, there is a problem in that, at a time of an inference, a difference in classification accuracy of the lesion occurs for each position in the organ region of the tomographic image input to the Segmentation model 60. As an example of the histogram 82, in a case where tomographic images of an upper portion and a lower portion in the organ region in the vertical direction are input, there is a possibility that the classification accuracy of the lesion is lowered.

On the other hand, the Classification model 70 classifies a lesion by using a patch image obtained by dividing a tomographic image as a unit. Since such a Classification model 70 classifies a lesion based on an image feature such as light and dark of an image, in a partial region in a tomographic image called a patch image, classification accuracy does not depend on a position of the patch image in an organ region.

Accordingly, the diagnosis support apparatus 100 according to the present embodiment selects and uses one of the Segmentation model 60 and the Classification model 70 at a time of an inference in accordance with the number of pieces of data in the organ region of the learning data of the lesion region, which is used at a time of training of the Segmentation model 60. For example, when a tomographic image of a certain division region in an organ region is set as an classification target, in a case where the number of pieces of learning data of a lesion region in the division region is large, lesion classification is executed by using the Segmentation model 60. On the other hand, in a case where the number of pieces of learning data of the lesion region in the division region is small, the lesion classification is executed by using the Classification model 70. Thus, it is possible to reduce a deterioration in classification accuracy of a lesion in a division region in which the number of pieces of learning data of a lesion region is small, and to classify the lesion with stable accuracy regardless of a position in an organ region.

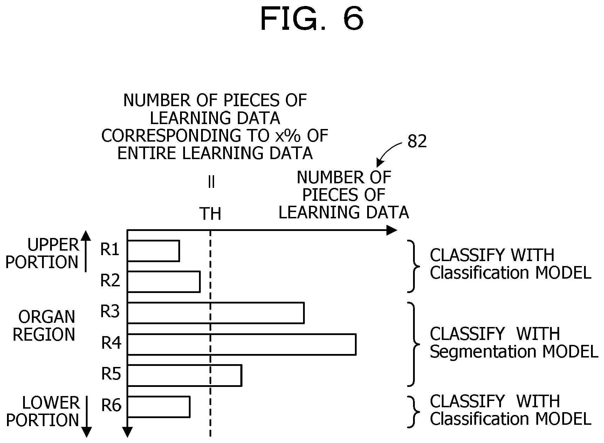

FIG. 6 is a diagram illustrating an example of selecting a lesion classification model. As illustrated in FIG. 6, a threshold value TH is set for the number of pieces of learning data of a lesion region for each division region. As the threshold value TH, for example, the number of pieces of learning data at a predetermined ratio (x%) with respect to a total number of pieces of learning data included in the learning data set 81 is set. In this case, when the diagnosis support apparatus 100 executes lesion classification for a tomographic image included in a certain division region, in a case where the number of pieces of learning data of a lesion region corresponding to the division region is more than the threshold value TH, the diagnosis support apparatus 100 executes the lesion classification by using the Segmentation model 60. On the other hand, in a case where the number of pieces of learning data of the lesion region is equal to or less than the threshold value TH, the diagnosis support apparatus 100 executes the lesion classification by using the Classification model 70.

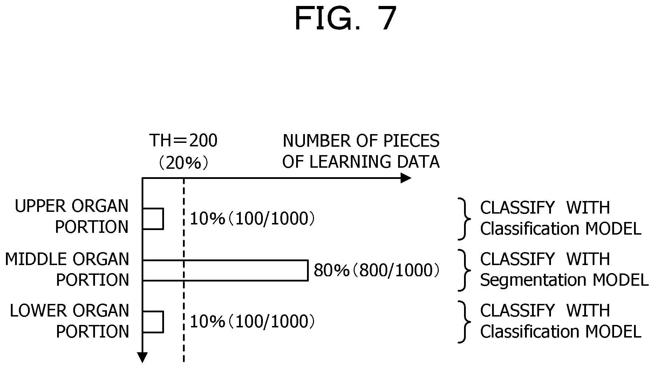

FIG. 7 is a diagram illustrating a specific example of model selection in accordance with the number of pieces of learning data of a lesion region. In the example illustrated in FIG. 7, an organ region is divided into three regions of "upper organ portion", "middle organ portion", and "lower organ portion" in the vertical direction. A total number of pieces of learning data included in the learning data set 81 is 1000, and the threshold value TH is set to 200 corresponding to 20% of the total number.

In the example illustrated in FIG. 7, it is assumed that among the pieces of learning data included in the learning data set 81, the number of pieces of learning data corresponding to the upper organ portion is 100. In this case, since the number of pieces of learning data is equal to or less than the threshold value TH, lesion classification for a tomographic image corresponding to the upper organ portion is executed by using the Classification model 70.

It is assumed that among the pieces of learning data included in the learning data set 81, the number of pieces of learning data corresponding to the middle organ portion is 800. In this case, since the number of pieces of learning data is more than the threshold value TH, lesion classification for a tomographic image corresponding to the middle organ portion is executed by using the Segmentation model 60.

It is assumed that among the pieces of learning data included in the learning data set 81, the number of pieces of learning data corresponding to the lower organ portion is 100. In this case, since the number of pieces of learning data is equal to or less than the threshold value TH, lesion classification for a tomographic image corresponding to the upper organ portion is executed by using the Classification model 70.

With such a process, it is possible to reduce a deterioration in classification accuracy of a lesion in a division region in which the number of pieces of learning data of a lesion region is small. As a result, the lesion may be classified with stable accuracy regardless of the position in the organ region in the tomographic image of the classification target.

Noted that as described in the examples in FIGs. 6 and 7, the threshold value TH may be set as an absolute number of pieces of learning data itself, for example, in addition to a value indicating a ratio to the total number of pieces of learning data. For example, depending on a lesion as an classification target or a corresponding disease, there may be a case where classification accuracy equal to or higher than a certain level is obtained when a certain number or more of pieces of learning data of a lesion region are secured. In such a case, the threshold value TH may be the minimum number of pieces of learning data of the lesion region desired for obtaining the certain or higher level of classification accuracy.

A division direction of the organ region for generating the division regions is not limited to the vertical direction as described above. For example, the organ region may be equally divided in a direction perpendicular to the slice plane of the tomographic image (parallel direction of the tomographic image).

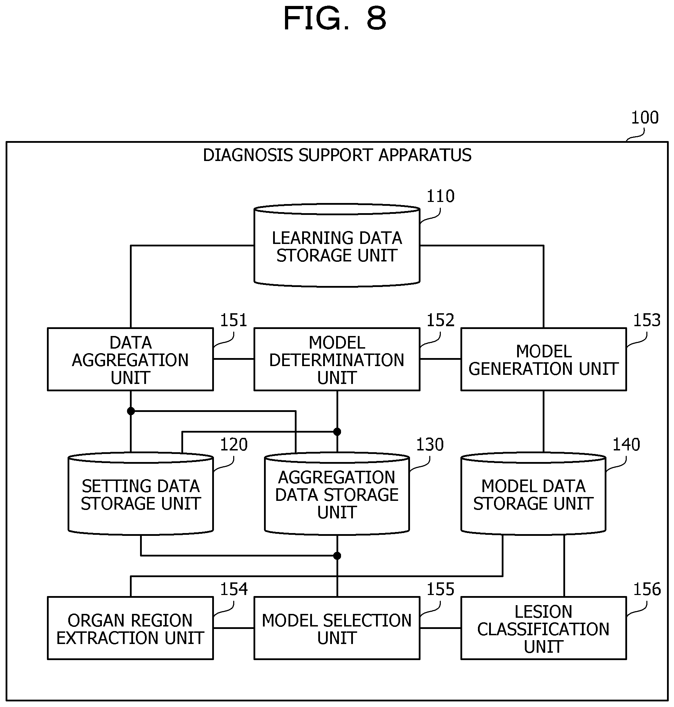

FIG. 8 is a diagram illustrating a configuration example of the processing function provided by the diagnosis support apparatus. The diagnosis support apparatus 100 includes a learning data storage unit 110, a setting data storage unit 120, an aggregation data storage unit 130, a model data storage unit 140, a data aggregation unit 151, a model determination unit 152, a model generation unit 153, an organ region extraction unit 154, a model selection unit 155, and a lesion classification unit 156.

The learning data storage unit 110, the setting data storage unit 120, the aggregation data storage unit 130, and the model data storage unit 140 are storage regions secured in a storage device included in the diagnosis support apparatus 100, such as the RAM 102 or the HDD 103.

The learning data storage unit 110 stores learning data to be used when the Segmentation model 60 and the Classification model 70 are generated by machine learning.

Setting data referred to in a process of determining a lesion classification model to be used for each division region is stored in the setting data storage unit 120.

Among the pieces of learning data stored in the learning data storage unit 110, the aggregation data storage unit 130 stores aggregation data related to learning data of a lesion region (learning data including a tomographic image including the lesion region as teaching data). As the aggregation data, the number of pieces of learning data of the lesion region for each of the division regions obtained by dividing an organ region is stored. For each of the division regions, the aggregation data storage unit 130 also stores information indicating which of the Segmentation model 60 and the Classification model 70 is to be used.

A model parameter corresponding to each of an organ region extraction model that is a trained model for extracting an organ region, the Segmentation model 60, and the Classification model 70 is stored in the model data storage unit 140.

The processes of the data aggregation unit 151, the model determination unit 152, the model generation unit 153, the organ region extraction unit 154, the model selection unit 155, and the lesion classification unit 156 are implemented, for example, by the processor 101 executing a predetermined program.

The data aggregation unit 151, the model determination unit 152, and the model generation unit 153 operate in a learning phase in which the Segmentation model 60 and the Classification model 70 are generated by machine learning.

Among the pieces of learning data stored in the learning data storage unit 110, the data aggregation unit 151 counts the number of pieces of learning data of the lesion region for each division region, and registers the counting result in the aggregation data storage unit 130.

Based on the number of pieces of learning data for each division region registered in the aggregation data storage unit 130, the model determination unit 152 determines which one of the Segmentation model 60 and the Classification model 70 is to be used for each division region, and registers the determination result in the aggregation data storage unit 130.

By performing machine learning using the learning data stored in the model data storage unit 140, the model generation unit 153 generates the Segmentation model 60 and the Classification model 70, and registers a model parameter indicating the generated each lesion classification model in the learning data storage unit 110.

The organ region extraction unit 154, the model selection unit 155, and the lesion classification unit 156 operate in an inference phase for classifying a lesion by using the Segmentation model 60 and the Classification model 70.

The organ region extraction unit 154 extracts a tomographic image included in an organ region from a tomographic image set obtained by imaging a diagnosis target person by using the organ region extraction model.

For each of the tomographic images extracted by the organ region extraction unit 154, the model selection unit 155 selects any one of the Segmentation model 60 and the Classification model 70 as a lesion classification model to be used for lesion classification, based on information registered in the aggregation data storage unit 130.

By using the lesion classification model selected by the model selection unit 155, the lesion classification unit 156 classifies whether or not each pixel or each patch image is a lesion region from the tomographic image.

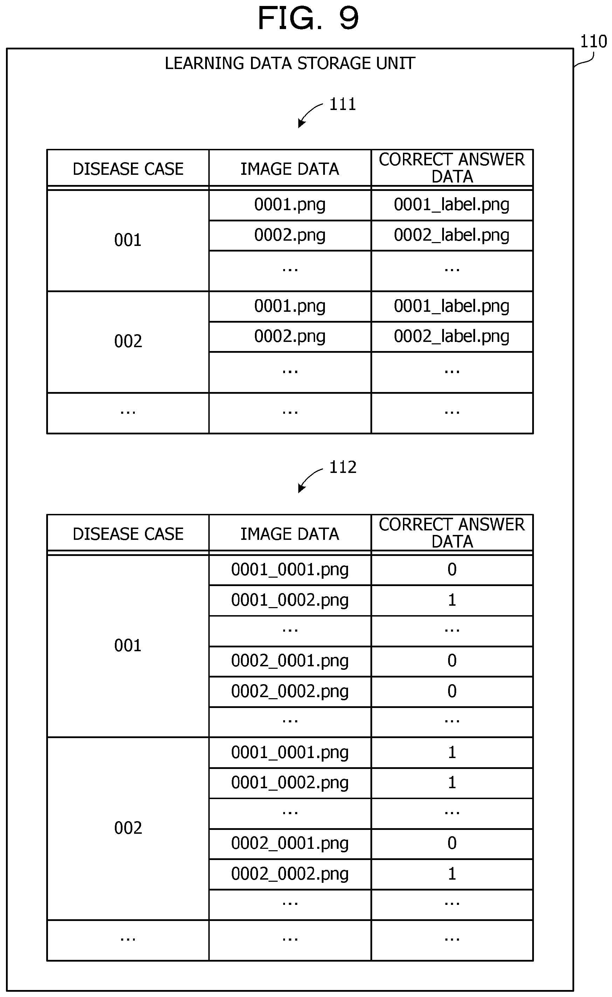

FIG. 9 is a diagram illustrating an example of data stored in a learning data storage unit. Learning data tables 111 and 112 are stored in the learning data storage unit 110.

Learning data to be used to generate the Segmentation model 60 is registered in the learning data table 111. For each of a plurality of disease cases, a plurality of sets of learning data including image data and correct answer data are registered in the learning data table 111. The image data is data of a tomographic image, and an image data group corresponding to the disease case is an image data group of a plurality of tomographic images each obtained by one imaging for one person. The correct answer data is data in which a label indicating whether or not each pixel of the corresponding tomographic image is a lesion is associated with the pixel.

Learning data to be used to generate the Classification model 70 are registered in the learning data table 112. For each of the plurality of disease cases, a plurality of sets of learning data including image data and correct answer data as teaching data are registered in the learning data table 112. The image data is data of patch images obtained by dividing a tomographic image into predetermined sizes, and an image data group corresponding to the disease case is an image data group of patch images obtained from each of a plurality of tomographic images obtained by one imaging for one person. The correct answer data is a label indicating whether or not the corresponding patch image is a lesion.

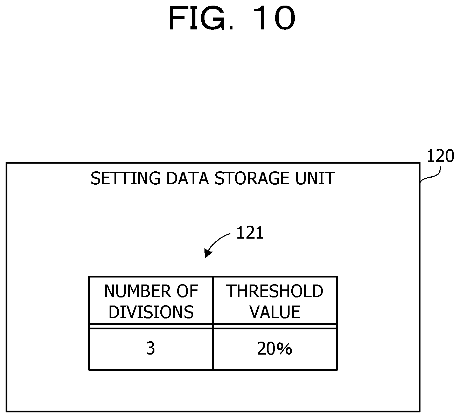

FIG. 10 is a diagram illustrating an example of data stored in a setting data storage unit. As described above, setting data referred to in a process of determining a lesion classification model to be used for each division region is stored in the setting data storage unit 120. As such data, a setting data set 121 including the number of divisions and a threshold value is stored in the setting data storage unit 120.

The number of divisions indicates the number of divisions of an organ region (for example, the number of divided division regions). The threshold value is a value indicating a ratio of a threshold value (the threshold value TH described above) used to determine which lesion classification model is to be used to the total number of pieces of learning data for a lesion region. The number of divisions and the threshold value may be freely set by a user.

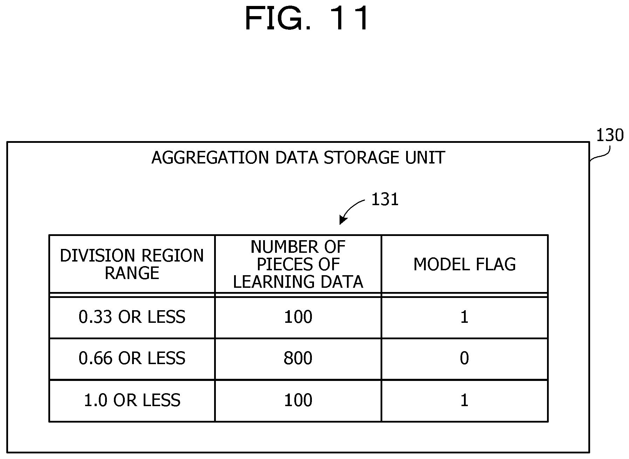

FIG. 11 is a diagram illustrating an example of data stored in an aggregation data storage unit. An aggregation data table 131 is stored in the aggregation data storage unit 130. A record for each division region is registered in the aggregation data storage unit 130. Each record includes a division region range, the number of pieces of learning data, and a model flag.

The division region range is a value indicating a range of a division region in an organ region with a ratio based on an upper end of the organ region. In the example of FIG. 11, the range of the division region is indicated by a ratio in which the upper end of the organ region is set to 0 and a lower end is set to 1. For example, in a case where the organ region is divided into three regions as illustrated in FIG. 11, a division region range of a first division region is 0 or more and 0.33 or less, a division region range of a second division region is more than 0.33 and 0.66 or less, and a division region range of a third division region is more than 0.66 and 1.0 or less.

The number of pieces of learning data indicates the number of pieces of learning data including a tomographic image included in a corresponding division region among pieces of learning data of a lesion region used when the Segmentation model 60 is generated.

The model flag is a flag indicating which of the Segmentation model 60 and the Classification model 70 is to be used when a lesion is classified for the tomographic image included in the corresponding division region. According to the present embodiment, it is assumed that the flag is set to 0 in a case where the Segmentation model 60 is to be used, and the flag is set to 1 in a case where the Classification model 70 is to be used.

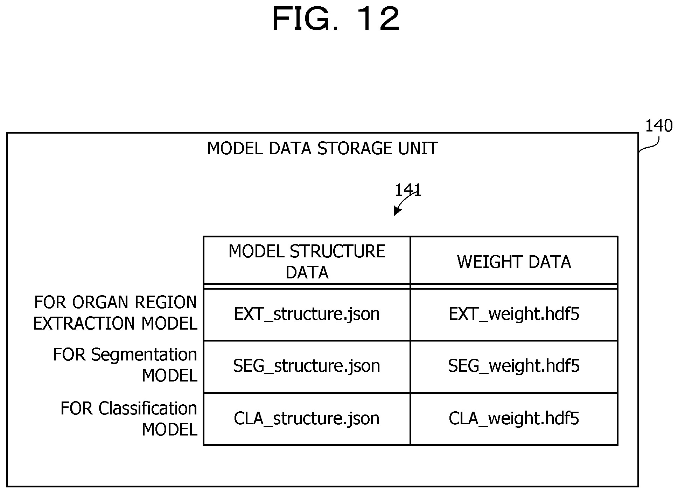

FIG. 12 is a diagram illustrating an example of data stored in a model data storage unit. A model data table 141 is stored in the model data storage unit 140. Model structure data and weight data are registered in the model data table 141 for each of the organ region extraction model, the Segmentation model 60, and the Classification model 70.

The model structure data indicates a type of a machine learning model to be used and a configuration of a neural network. A file name of a file in which such information is described may be registered in the model data table 141 in practice. The weight data indicates a weight coefficient set between nodes in the neural network of the machine learning model. Actually, a file name of a file in which such a weight coefficient is described may be registered in the model data table 141.

The model structure data and the weight data for the organ region extraction model are registered in the model data table 141 in advance. Each model structure data for the Segmentation model 60 and the Classification model 70 is also registered in the model data table 141 in advance. On the other hand, each weight data for the Segmentation model 60 and the Classification model 70 is generated by the model generation unit 153, is registered in the model data table 141.

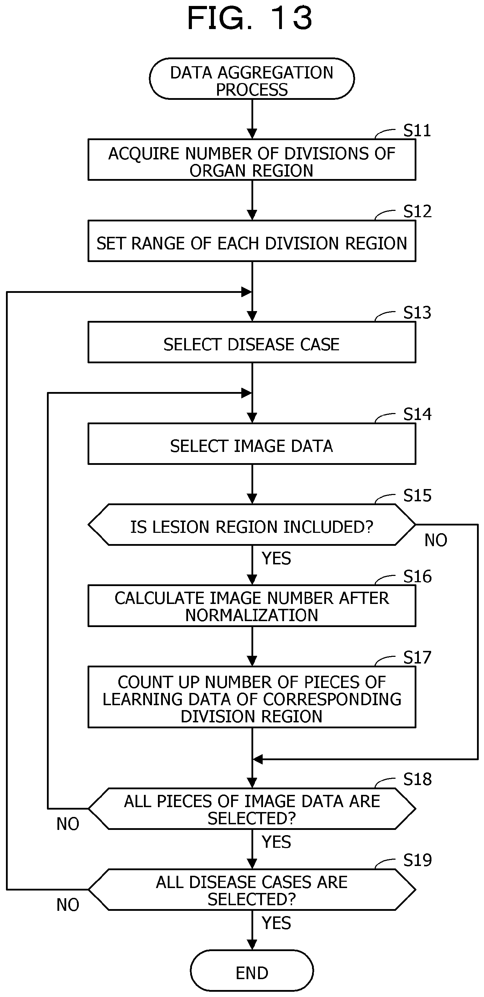

Next, a process of the diagnosis support apparatus 100 will be described with reference to a flowchart. First, a process in a learning phase will be described with reference to FIGs. 13 to 15.

FIG. 13 is an example of a flowchart illustrating a procedure of a data aggregation process.

[step S11] The data aggregation unit 151 acquires the number of divisions of an organ region from the setting data set 121 in the setting data storage unit 120.

[step S12] The data aggregation unit 151 creates records for the acquired number of divisions, in the aggregation data table 131 of the aggregation data storage unit 130, and sets a range (division region range) of each division region. Assuming that the number of divisions is d, the division region ranges are, in order from an upper side of the body, a range of 0 or more and 1/d or less, a range of more than 1/d and 2/d or less, and ..., a range of more than d-1/d and 1 or less. The data aggregation unit 151 sets an initial value of 0 as the number of pieces of learning data of each record created in the aggregation data table 131.

[step S13] The data aggregation unit 151 selects one disease case from the learning data table 111 for the Segmentation model 60 stored in the learning data storage unit 110.

The data aggregation unit 151 assigns an image number starting from 0 to image data of a tomographic image included in the selected disease case, in order from image data at an upper end of the body. Among these pieces of image data, the data aggregation unit 151 sets an image number of the image data at the upper end of the organ region to Iₘᵢₙ, and sets an image number of the image data at a lower end of the organ region to Iₘₐₓ.

Since all the tomographic images registered as the learning data are images in the organ region, Iₘᵢₙ = 0 and Iₘₐₓ = Iₘᵢₙ + (the number of pieces of image data included in the selected disease case) - 1 are set in step S13.

[step S14] The data aggregation unit 151 selects one piece of image data included in the selected disease case. With this process, among the pieces of image data included in the disease case, one piece of unselected image data is selected sequentially from a head side (upper side).

[step S15] The data aggregation unit 151 determines whether or not the selected image data includes a lesion region. With this process, the data aggregation unit 151 acquires correct answer data corresponding to the selected image data, from the learning data table 111. When one or more pixels having a label indicating a lesion exist in the acquired correct answer data, the data aggregation unit 151 determines that the selected image data includes the lesion region. When it is determined that the selected image data includes the lesion region, the process proceeds to step S16, and when it is determined that the selected image data does not include the lesion region, the process proceeds to step S18.

[step S16] In order to determine in which division region the selected image data is included, the data aggregation unit 151 calculates an image number x_{after} after normalization for the selected image data. Assuming that the image number of the selected image data is x, the image number x_{after} is calculated by the following Equation (1). x_{after} = (x - Iₘᵢₙ)/(Iₘₐₓ - Iₘᵢₙ) ... (1)

According to Equation (1), the image number x is normalized to a value equal to or more than 0 and equal to or less than 1. For example, the image number x_{after} calculated by Equation (1) indicates a ratio position of the selected image data in the entire organ region from the head side.

[step S17] The data aggregation unit 151 determines in which division region range set in the aggregation data table 131 in step S12 the calculated image number xₐᵣₜₑᵣ is included. Thus, the data aggregation unit 151 determines a division region to which the selected image data belongs. Among the numbers of pieces of learning data in the aggregation data table 131, the data aggregation unit 151 counts up the number of pieces of learning data corresponding to the division region to which the selected image data belongs by 1.

[step S18] The data aggregation unit 151 determines whether all the image data included in the disease case selected in step S13 are selected. In a case where there is unselected image data, the process proceeds to step S14, and the image data on the head side among the unselected image data is selected. On the other hand, in a case where all the corresponding pieces of image data are selected, the process proceeds to step S19.

[step S19] The data aggregation unit 151 determines whether all the disease cases included in the learning data table 111 are selected. In a case where there is an unselected disease case, the process proceeds to step S13, and one of the unselected disease cases is selected. On the other hand, in a case where all the disease cases are selected, the data aggregation process ends.

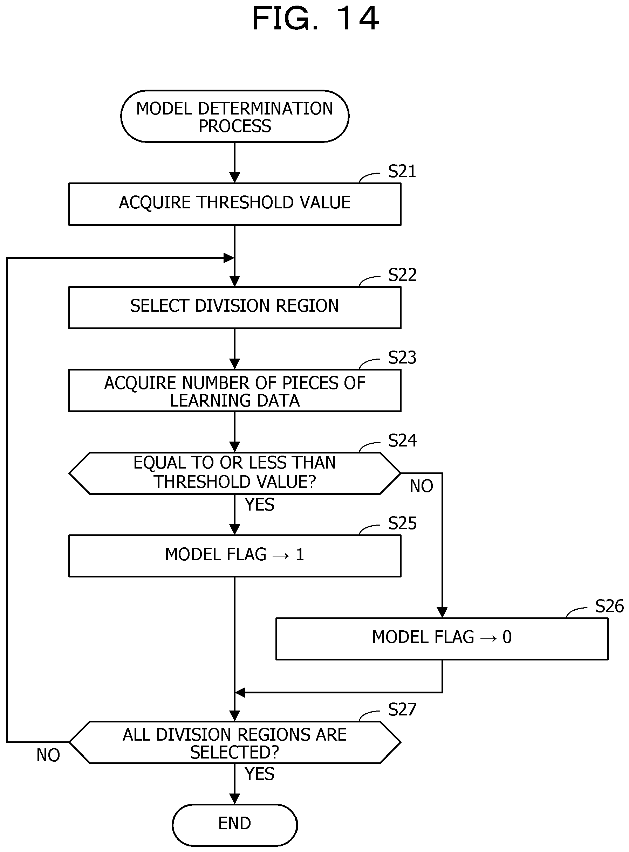

With the process described above, the number of pieces of learning data for each division region is registered in the aggregation data table 131. By referring to the aggregation data table 131, the model determination process illustrated in FIG. 14 is executed.

FIG. 14 is an example of a flowchart illustrating a procedure of a model determination process.

[step S21] The model determination unit 152 acquires a threshold value from the setting data set 121 in the setting data storage unit 120.

[step S22] The model determination unit 152 selects one of the division regions of which division region ranges are set in the aggregation data table 131 of the aggregation data storage unit 130.

[step S23] From the aggregation data table 131, the model determination unit 152 acquires the number of pieces of learning data corresponding to the selected division region.

[step S24] The model determination unit 152 determines whether or not the acquired number of pieces of learning data is equal to or less than the threshold value acquired in step S21. In a case where the number of pieces of learning data is equal to or less than the threshold value, the process proceeds to step S25, and in a case where the number of pieces of learning data is more than the threshold value, the process proceeds to step S26.

Noted that in a case where the threshold value registered in the setting data set 121 is a value indicating a ratio, the model determination unit 152 converts this threshold value into an absolute number of pieces of learning data, and compares the converted threshold value with the acquired number of pieces of learning data. Specifically, the model determination unit 152 sums up the numbers of pieces of learning data of the respective division regions registered in the aggregation data table 131, and multiplies the total value by a threshold value indicating a ratio to convert the total value into a threshold value indicating the absolute number of pieces of learning data.

[step S25] The model determination unit 152 sets 1 as a model flag corresponding to the selected division region in the aggregation data table 131. Thus, it is set that the Classification model 70 is to be used for lesion classification of a tomographic image included in the selected division region.

[step S26] For the aggregation data table 131, the model determination unit 152 sets 0 as the model flag corresponding to the selected division region. Thus, it is set that the Segmentation model 60 is to be used for the lesion classification of the tomographic image included in the selected division region.

[step S27] The model determination unit 152 determines whether all the division regions are selected. In a case where there is an unselected division region, the process proceeds to step S22, and one of the unselected division regions is selected. On the other hand, in a case where all the division regions are selected, the model determination process ends.

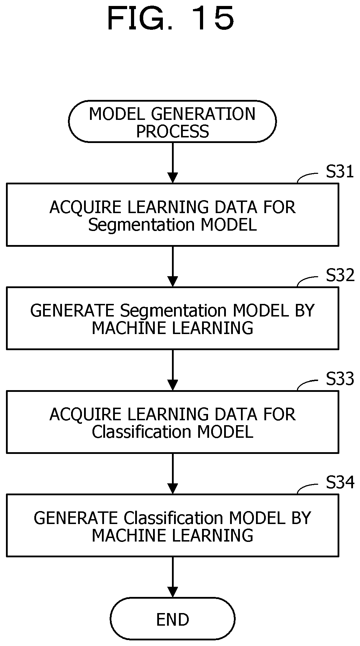

FIG. 15 is an example of a flowchart illustrating a procedure of a model generation process.

[step S31] The model generation unit 153 acquires learning data for the Segmentation model 60 from the learning data table 111.

[step S32] By executing machine learning using the acquired learning data, the model generation unit 153 generates the Segmentation model 60. Specifically, the model generation unit 153 calculates weight data of the Segmentation model 60 by machine learning, and registers the weight data in a record corresponding to the Segmentation model 60 among records included in the model data table 141.

[step S33] The model generation unit 153 acquires learning data for the Classification model 70 from the learning data table 112.

[step S34] By executing machine learning using the acquired learning data, the model generation unit 153 generates the Classification model 70. Specifically, the model generation unit 153 calculates weight data of the Classification model 70 by machine learning, and registers the calculated weight data in a record corresponding to the Classification model 70 among the records included in the model data table 141.

Noted that the model generation process in FIG. 15 may be executed after the processes in FIGs. 13 and 14, or may be executed before the processes in FIGs. 13 and 14.

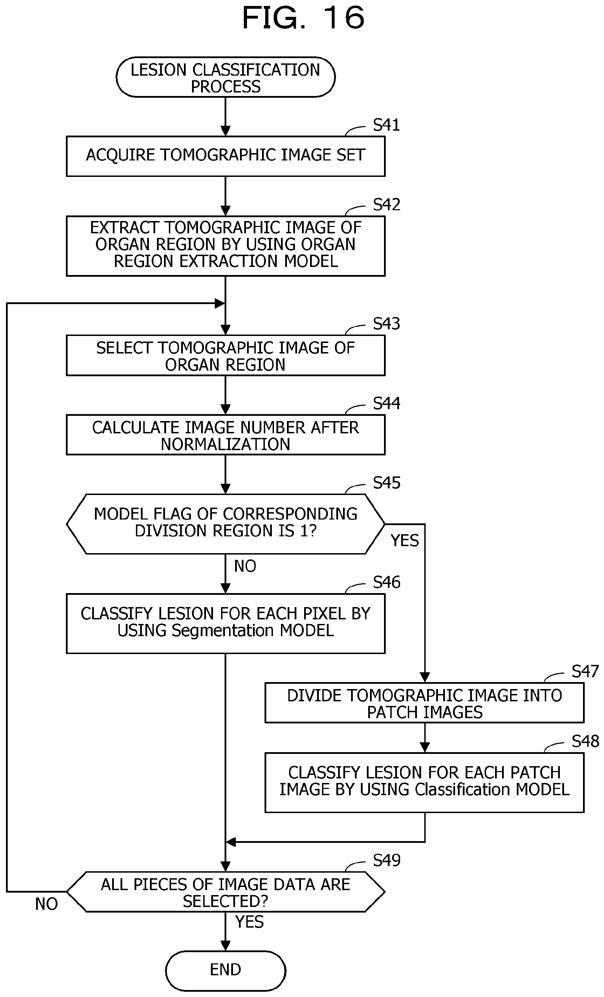

Next, a process in an inference phase will be described with reference to FIG. 16.

FIG. 16 is an example of a flowchart illustrating a procedure of a lesion classification process.

[step S41] The organ region extraction unit 154 acquires a tomographic image set of a diagnosis target person, which is captured by the CT apparatus 50. The organ region extraction unit 154 assigns an image number starting from 1 to each tomographic image included in the acquired tomographic image set, in order from an upper side of the body.

[step S42] The organ region extraction unit 154 inputs the acquired tomographic image set to the organ region extraction model based on the weight data registered in the model data table 141. Thus, the organ region extraction unit 154 extracts a tomographic image included in an organ region among the tomographic images included in the tomographic image set.

Among the extracted tomographic images, the model selection unit 155 sets the image number of a head (upper end) tomographic image to Iₘᵢₙ, and sets the image number of a last (lower end) tomographic image to Iₘₐₓ.

[step S43] The model selection unit 155 selects one tomographic image of the organ region. With this process, unselected tomographic images are selected in order from the head side, among the tomographic images extracted in step S42.

[step S44] In order to determine in which division region the selected tomographic image is included, the model selection unit 155 calculates the image number x_{after} after normalization for the selected image data. Assuming that the image number of the selected tomographic image is x, the image number x_{after} is calculated by Equation (1) described above. Thus, a ratio position of the selected tomographic image from the head side in the entire organ region is calculated.

[step S45] Among the division region ranges registered in the aggregation data table 131, the model selection unit 155 determines in which division region range the calculated image number x_{after} is included. Thus, the model selection unit 155 determines a division region to which the selected tomographic image belongs.

Among the model flags in the aggregation data table 131, the model selection unit 155 acquires a model flag corresponding to the division region to which the selected tomographic image belongs, and determines whether the acquired model flag is 1. In a case where the model flag is 0, the process proceeds to step S46, and in a case where the model flag is 1, the process proceeds to step S47.

[step S46] The lesion classification unit 156 inputs the tomographic image selected in step S43 to the Segmentation model 60 based on the weight data registered in the model data table 141. Thus, the lesion classification unit 156 classifies whether or not each pixel in the tomographic image is a lesion.

[step S47] The lesion classification unit 156 divides the tomographic image selected in step S43 into patch images having a certain size.

[step S48] The lesion classification unit 156 inputs the divided patch images one by one to the Classification model 70 based on the weight data registered in the model data table 141. Thus, the lesion classification unit 156 classifies whether or not each patch image over the tomographic image is a lesion.

[step S49] The lesion classification unit 156 determines whether all tomographic images in the organ region are selected. In a case where there is an unselected tomographic image, the process proceeds to step S43, and one of the unselected tomographic images is selected. On the other hand, in a case where all the corresponding tomographic images are selected, the lesion classification process ends.

Noted that for example, in steps S46 and S48, the lesion classification unit 156 generates an classification result image to which information indicating whether or not each pixel of the selected tomographic image is a lesion is assigned. After that, in a case where a display of an classification result of the tomographic image is requested, the lesion classification unit 156 displays a region determined to have a lesion over the tomographic image in a manner distinguished from other regions based on the classification result image.

As described above, the Segmentation model 60 and the Classification model 70 may be lesion classification models for classifying three or more types of lesions including a type indicating that the model is not a lesion. In this case, in steps S46 and S48, for example, an classification result image to which information indicating one of the lesion types is assigned is generated for each pixel of the tomographic image.

With the process described above, it is possible to reduce a deterioration in classification accuracy of a lesion in a division region in which the number of pieces of learning data of a lesion region is small. As a result, the lesion may be classified with stable accuracy regardless of the position in the organ region in the tomographic image of the classification target.

Noted that the processes in FIGs. 13 to 15 in the learning phase and the process in FIG. 16 in the inference phase may be executed in different information processing apparatuses. In this case, for example, the learning data storage unit 110, the setting data storage unit 120, the aggregation data storage unit 130, the model data storage unit 140, the data aggregation unit 151, the model determination unit 152, and the model generation unit 153 are implemented in a first information processing apparatus. On the other hand, the organ region extraction unit 154, the model selection unit 155, and the lesion classification unit 156 are implemented in a second information processing apparatus. Data stored in the setting data storage unit 120, the aggregation data storage unit 130, and the model data storage unit 140 is transferred to the second information processing apparatus via a network or a portable-type recording medium, and these pieces of data are referred to in the process in the inference phase.

### [Third Embodiment]

According to the second embodiment described above, the lesion classification model is selected depending on whether or not the number of pieces of learning data of the lesion region used at the time of training is large for the division region to which the captured tomographic image belongs. Meanwhile, in a third embodiment, when the Segmentation model 60 is generated, the classification accuracy of the Segmentation model 60 is verified for each of the division regions. In an inference phase, the lesion classification model is selected depending on whether or not the classification accuracy of the Segmentation model 60 is high for the division region to which the captured tomographic image belongs.

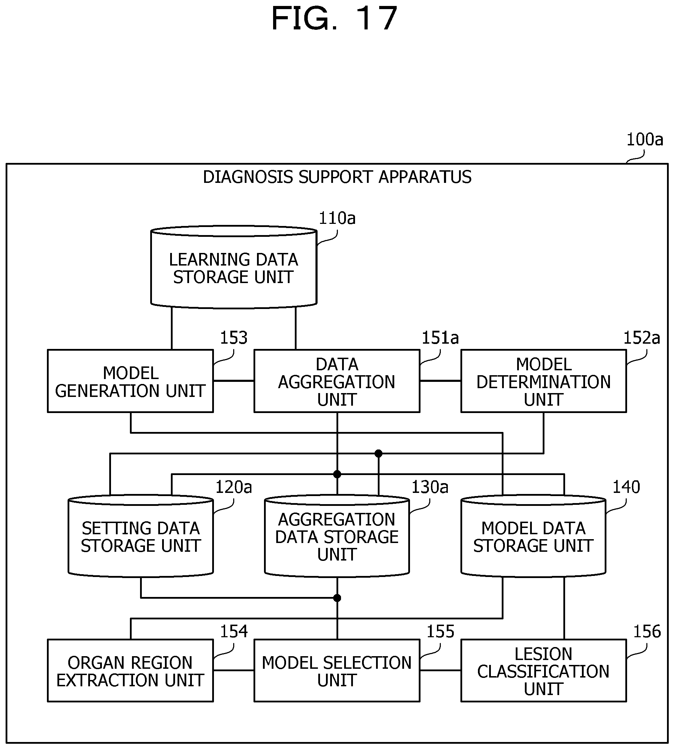

FIG. 17 is a diagram illustrating a configuration example of a processing function provided by a diagnosis support apparatus according to the third embodiment. In FIG. 17, the same components as the components illustrated in FIG. 8 are indicated by the same reference signs, and will not be described.

A diagnosis support apparatus 100a according to the third embodiment includes a learning data storage unit 110a, a setting data storage unit 120a, and an aggregation data storage unit 130a, instead of the learning data storage unit 110, the setting data storage unit 120, and the aggregation data storage unit 130 illustrated in FIG. 8.

In the same manner as the learning data storage unit 110, the learning data storage unit 110a includes learning data to be used when the Segmentation model 60 is generated and learning data to be used when the Classification model 70 is generated. A part of the former learning data is used for verifying classification accuracy of the Segmentation model 60.

The setting data storage unit 120a is different from the setting data storage unit 120 in that a value of the classification accuracy is set as a threshold value.

Instead of the number of pieces of learning data, the number of verifications and the number of correct answers at a time of verifying the classification accuracy of the Segmentation model 60 are registered in the aggregation data storage unit 130a.

The diagnosis support apparatus 100a includes a data aggregation unit 151a and a model determination unit 152a, instead of the data aggregation unit 151 and the model determination unit 152 illustrated in FIG. 8.

After the Segmentation model 60 is generated by the model generation unit 153, the data aggregation unit 151a verifies the classification accuracy of the Segmentation model 60 for each of the division regions by using the learning data for verification registered in the learning data storage unit 110a.

The model determination unit 152a determines a lesion classification model to be used for each of the division regions based on a result of verification by the data aggregation unit 151a.

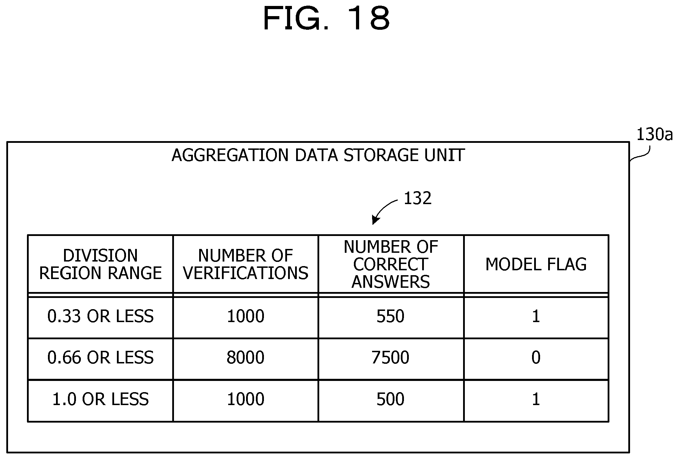

FIG. 18 is a diagram illustrating an example of data stored in an aggregation data storage unit. An aggregation data table 132 is stored in the aggregation data storage unit 130a. A record for each division region is registered in the aggregation data table 132. Each record includes a division region range, the number of verifications, the number of correct answers, and a model flag.

The division region range and the model flag are the same as the division region range and the model flag in the aggregation data table 131 illustrated in FIG. 11. The number of verifications indicates the number of tomographic images (or the number of pixels) for which classification accuracy is verified by using the Segmentation model 60. The number of correct answers indicates the number of tomographic images (or the number of pixels) determined to be correct by the verification.

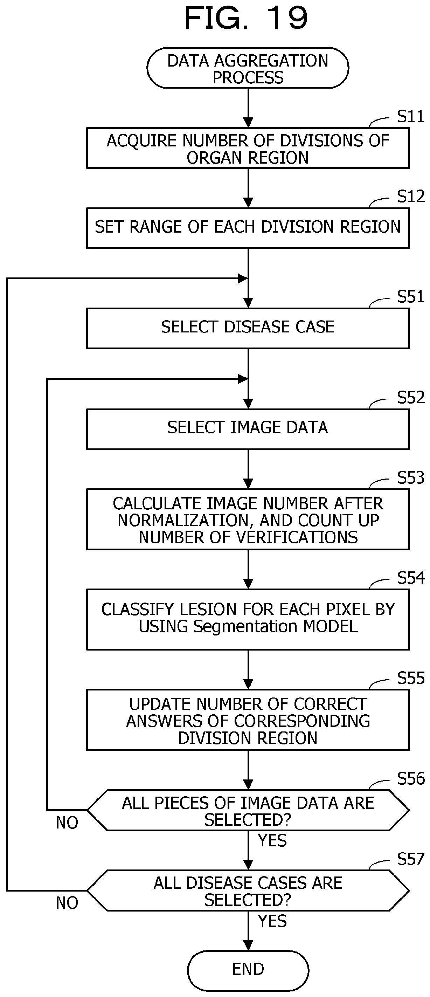

FIG. 19 is another example of the flowchart illustrating the procedure of the data aggregation process.

The data aggregation unit 151a executes the processes in steps S11 and S12 in FIG. 13. In step S12, a record is created for the aggregation data table 132. Next, the process in and after next step S51 is executed.

[step S51] The data aggregation unit 151a selects one disease case for verification from a learning data table for the Segmentation model 60 stored in a learning data storage unit 110a.

The data aggregation unit 151 assigns an image number starting from 0 to image data of a tomographic image included in the selected disease case, in order from image data at an upper end of the body. Among these pieces of image data, the data aggregation unit 151 sets an image number of the image data at the upper end of the organ region to Iₘᵢₙ, and sets an image number of the image data at a lower end of the organ region to Iₘₐₓ.

Since all the tomographic images registered as the learning data are images in the organ region, Iₘᵢₙ = 0 and Iₘₐₓ = Iₘᵢₙ + (the number of pieces of image data included in the selected disease case) - 1 are set in step S51.

[step S52] The data aggregation unit 151a selects one piece of image data included in the selected disease case. With this process, among the pieces of image data included in the disease case, one piece of unselected image data is selected sequentially from a head side (upper side).

[step S53] In order to determine in which division region the selected image data is included, the data aggregation unit 151a calculates the image number x_{after} after normalization for the selected image data. Assuming that the image number of the selected image data is x, the image number x_{after} is calculated by Equation (1) described above. Thus, a ratio position of the selected image data from the head side in the entire organ region is calculated.

The data aggregation unit 151a determines in which division region range set in the aggregation data table 132 in step S12 the calculated image number x_{after} is included. Thus, the data aggregation unit 151a determines a division region to which the selected image data belongs. Among the numbers of verifications in the aggregation data table 132, the data aggregation unit 151a counts up the number of verifications corresponding to the division region to which the selected image data belongs by 1.

[step S54] The data aggregation unit 151a inputs the image data selected in step S52 to the Segmentation model 60 based on the weight data registered in the model data table 141. Thus, the data aggregation unit 151a classifies whether or not each pixel over the tomographic image is a lesion.

[step S55] The data aggregation unit 151a compares an classification result for each pixel in step S54 with a value for each pixel of correct answer data corresponding to the selected image data. A case where the classification result and the pixel value coincide with each other indicates a correct answer. For example, in a case where the number of correct pixels among the pixels of the image data is equal to or more than a certain ratio, the data aggregation unit 151a determines that classification accuracy for the selected image data is high. In this case, among the numbers of correct answers in the aggregation data table 132, the data aggregation unit 151a counts up the number of correct answers corresponding to the division region to which the selected image data belongs by 1.

Noted that in step S53, the number of pixels of the image data may be added to the corresponding number of verifications registered in the aggregation data table 132. In this case, in step S55, the number of correct pixels may be added to the corresponding number of correct answers registered in the aggregation data table 132.

[step S56] The data aggregation unit 151a determines whether all the image data included in the disease case selected in step S51 are selected. In a case where there is unselected image data, the process proceeds to step S52, and the image data on the head side among the unselected image data is selected. On the other hand, in a case where all the corresponding pieces of image data are selected, the process proceeds to step S57.

[step S57] The data aggregation unit 151a determines whether all the disease cases for verification included in the learning data table 111 are selected. In a case where there is an unselected disease case, the process proceeds to step S51, and one of the unselected disease cases is selected. On the other hand, in a case where all the disease cases are selected, the data aggregation process ends.

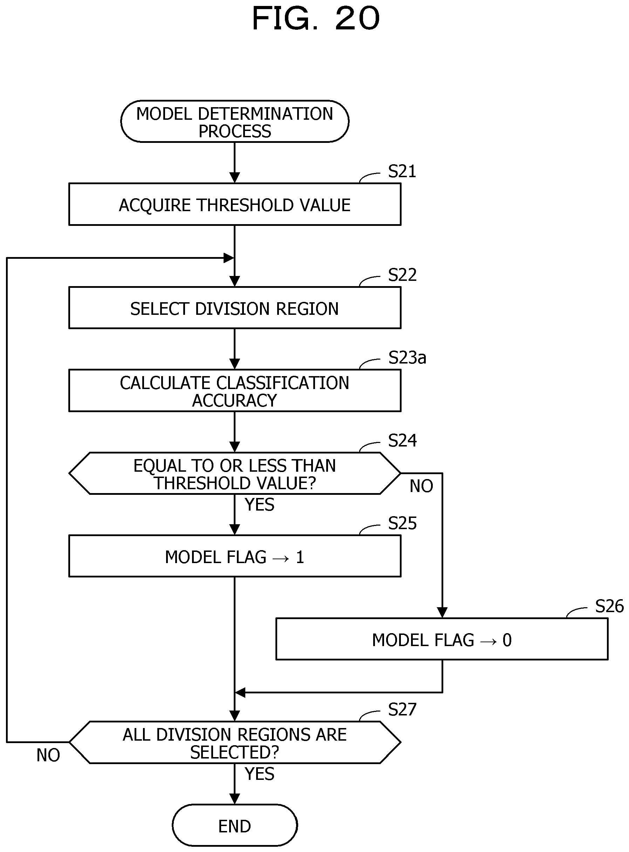

With the process described above, the number of verifications and the number of correct answers for each division region are registered in the aggregation data table 132. By referring to such an aggregation data table 132, the model determination process illustrated in FIG. 20 is executed.

FIG. 20 is an example of a flowchart illustrating a procedure of a model determination process.

In the process in FIG. 20, the processes in steps S21, S22, and S24 to S27 in FIG. 14 are executed by the model determination unit 152a. Instead of step S23 in FIG. 14, the following step S23a is executed.

[step S23a] The model determination unit 152a acquires the number of verifications and the number of correct answers from a record corresponding to the selected division region, among records in the aggregation data table 132. The model determination unit 152a calculates classification accuracy corresponding to the selected division region by a ratio of the number of correct answers to the number of verifications.

In next step S24, the calculated classification accuracy is compared with a threshold value.

According to the third embodiment described above, a lesion may be classified with stable accuracy, regardless of a position in an organ region in a tomographic image of an classification target.

Noted that the processing function of the apparatus (for example, the lesion detection apparatus 1 and the diagnosis support apparatuses 100 and 100a) described in each of the embodiments described above may be implemented by a computer. In such a case, a program describing the processing contents of the functions to be included in each apparatus is provided, and the processing functions described above are implemented over the computer by executing the program with the computer. The program describing the processing contents may be recorded in a computer-readable recording medium. Examples of the computer-readable recording medium include a magnetic storage device, an optical disc, a semiconductor memory, and the like. Examples of the magnetic storage device include a hard disk drive (HDD), a magnetic tape, and the like. Examples of the optical disc include a compact disc (CD), a Digital Versatile Disc (DVD), a Blu-ray disc (BD, registered trademark), and the like.

In a case where the program is distributed, for example, a portable-type recording medium such as a DVD or a CD on which the program is recorded is sold. The program may be stored in a storage device of a server computer and transferred from the server computer to another computer via a network.

The computer that executes the program stores, in a storage device thereof, the program recorded on the portable-type recording medium or the program transferred from the server computer, for example. The computer reads the program from the storage device thereof and executes the processing according to the program. Noted that the computer may also read the program directly from the portable-type recording medium and execute the processing according to the program. Each time the program is transferred from the server computer coupled to the computer via the network, the computer may also sequentially execute the processing according to the received program.

## Claims

1. A lesion detection method executed by a computer, the method comprising:
acquiring a first tomographic image (22a) obtained by imaging a first imaging position of an inside of a first human body (22);
selecting any one of a first machine learning model (2a) that classifies whether or not a pixel included in an input tomographic image (22a) is a specific lesion region in units of the pixel and a second machine learning model (2b) that classifies whether or not an image block obtained by dividing the input tomographic image (22a) into certain sizes is the specific lesion region in units of the image block, based on, among a plurality of tomographic images (22a) which are obtained by imaging an inside of one or more second human bodies (11), are used as learning data when the first machine learning model (2a) is generated by machine learning, and each of which includes the specific lesion region, the number of tomographic images (22a) captured at a second imaging position of the inside of the one or more second human bodies (11), which corresponds to the first imaging position; and
detecting the specific lesion region from the first tomographic image (22a) by using a machine learning model selected between the first machine learning model (2a) and the second machine learning model (2b);
wherein the second machine learning model (2b) classifies the lesion region based on a feature in a division region (A1-A3, B1-B3) instead of the entire tomographic image (22a).

2. The lesion detection method according to claim 1, further comprising:
counting the number of tomographic images (22a) captured in a corresponding first division region (A1-A3, B1-B3) among the plurality of tomographic images (22a), for each of a plurality of first division regions (A1-A3, B1-B3) obtained by dividing a predetermined organ region in the one or more second human bodies (11) into a predetermined number of divisions in a predetermined direction,
wherein in the selecting,
a third division region (A1-A3, B1-B3) is specified in which the first tomographic image (22a) is captured, among a plurality of second division regions (A1-A3, B1-B3) obtained by dividing the organ region in the first human body (22) into the predetermined number of divisions in the predetermined direction, and
any one of the first machine learning model and the second machine learning model (2b) is selected, based on the number of images counted for a fourth division region (A1-A3, B1-B3) which corresponds to the third division region (A1-A3, B1-B3) among the plurality of first division regions (A1-A3, B1-B3).

3. The lesion detection method according to claim 2,
wherein in the selecting, the first machine learning model (2a) is selected in a case where a ratio of the number of images counted for the fourth division region (A1-A3, B1-B3) to a total number of the plurality of tomographic images (22a) is more than a predetermined threshold value, and the second machine learning model (2b) is selected in a case where the ratio is equal to or less than the predetermined threshold value.

4. A lesion detection program causing a computer to execute a process comprising:
acquiring a first tomographic image obtained by imaging a first imaging position of an inside of a first human body (22);
selecting any one of a first machine learning model (2a) that classifies whether or not a pixel included in an input tomographic image (22a) is a specific lesion region in units of the pixel and a second machine learning model (2b) that classifies whether or not an image block obtained by dividing the input tomographic image (22a) into certain sizes is the specific lesion region in units of the image block, based on, among a plurality of tomographic images (22a) which are obtained by imaging an inside of one or more second human bodies (11), are used as learning data when the first machine learning model (2a) is generated by machine learning, and each of which includes the specific lesion region, the number of tomographic images (22a) captured at a second imaging position of the inside of the one or more second human bodies (11), which corresponds to the first imaging position; and
detecting the specific lesion region from the first tomographic image (22a) by using a machine learning model selected between the first machine learning model (2a) and the second machine learning model (2b);
wherein the second machine learning model (2b) classifies the lesion region based on a feature in a division region (A1-A3, B1-B3) instead of the entire tomographic image (22a).

## Patentansprüche

1. Läsionserkennungsverfahren, das von einem Computer ausgeführt wird, wobei das Verfahren umfasst:
Aufnehmen eines ersten tomographischen Bildes (22a), das durch Aufnahme einer ersten Bildaufnahmeposition eines Inneren eines ersten menschlichen Körpers (22) erhalten wird;
Auswählen eines aus einem ersten maschinellen Lernmodell (2a), das klassifiziert, ob ein in einem Eingabe-tomographischen Bild (22a) eingeschlossenes Pixel in Einheiten des Pixels ein spezifischer Läsionsbereich ist oder nicht, und einem zweiten maschinellen Lernmodell (2b), das klassifiziert, ob ein Bildblock, der durch Unterteilen des Eingabe-tomographischen Bildes (22a) in bestimmte Größen erhalten wird, in Einheiten des Bildblocks der spezifische Läsionsbereich ist oder nicht, auf der Grundlage von unter einer Vielzahl von tomographischen Bildern (22a), die durch Aufnahme eines Inneren eines oder mehrerer zweiter menschlicher Körper (11) erhalten werden, als Lerndaten verwendet werden, wenn das erste maschinelle Lernmodell (2a) durch maschinelles Lernen erzeugt wird, und von denen jedes den spezifischen Läsionsbereich einschließt, der Anzahl von tomographischen Bildern (22a), die an einer zweiten Bildaufnahmeposition des Inneren des einen oder der mehreren zweiten menschlichen Körper (11) aufgenommen werden, die der ersten Bildaufnahmeposition entspricht; und
Detektieren des spezifischen Läsionsbereichs aus dem ersten tomographischen Bild (22a) unter Verwendung von einem zwischen dem ersten maschinellen Lernmodell (2a) und dem zweiten maschinellen Lernmodell (2b) ausgewählten maschinellen Lernmodell;
wobei das zweite maschinelle Lernmodell (2b) den Läsionsbereich auf der Grundlage eines Merkmals in einem Unterteilungsbereich (A1-A3, B1-B3) anstelle des gesamten tomographischen Bildes (22a) klassifiziert.

2. Läsionserkennungsverfahren nach Anspruch 1, weiter umfassend:
Zählen der Anzahl von tomographischen Bildern (22a), die in einem entsprechenden ersten Unterteilungsbereich (A1-A3, B1-B3) unter der Vielzahl von tomographischen Bildern (22a) aufgenommen werden, für jeden einer Vielzahl von ersten Unterteilungsbereichen (A1-A3, B1-B3), die durch Unterteilen einer vorbestimmten Organregion in dem einen oder den mehreren zweiten menschlichen Körpern (11) in eine vorbestimmte Anzahl von Unterteilungen in einer vorbestimmten Richtung erhalten werden,
wobei beim Auswählen,
ein dritter Unterteilungsbereich (A1-A3, B1-B3) spezifiziert wird, in dem das erste tomographische Bild (22a) aufgenommen wird, unter einer Vielzahl von zweiten Unterteilungsbereichen (A1-A3, B1-B3), die durch Unterteilen der Organregion in dem ersten menschlichen Körper (22) in die vorbestimmte Anzahl von Unterteilungen in der vorbestimmten Richtung erhalten werden, und
eines aus dem ersten maschinellen Lernmodell und dem zweiten maschinellen Lernmodell (2b) auf der Grundlage von der Anzahl von Bildern ausgewählt wird, die für einen vierten Unterteilungsbereich (A1-A3, B1-B3) gezählt werden, der dem dritten Unterteilungsbereich (A1-A3, B1-B3) unter der Vielzahl von ersten Unterteilungsbereichen (A1-A3, B1-B3) entspricht.

3. Läsionserkennungsverfahren nach Anspruch 2,
wobei beim Auswählen das erste maschinelle Lernmodell (2a) in einem Fall ausgewählt wird, in dem ein Verhältnis der Anzahl von Bildern, die für den vierten Unterteilungsbereich (A1-A3, B1-B3) gezählt werden, zu einer Gesamtzahl der Vielzahl von tomographischen Bildern (22a) mehr als ein vorbestimmter Schwellenwert ist, und das zweite maschinelle Lernmodell (2b) in einem Fall ausgewählt wird, in dem das Verhältnis gleich dem vorbestimmten Schwellenwert oder kleiner als dieser ist.

4. Läsionserkennungsprogramm, das einen Computer veranlasst, ein Verfahren auszuführen, umfassend:
Aufnehmen eines ersten tomographischen Bildes, das durch Aufnahme einer ersten Bildaufnahmeposition eines Inneren eines ersten menschlichen Körpers (22) erhalten wird;
Auswählen eines aus einem ersten maschinellen Lernmodell (2a), das klassifiziert, ob ein in einem Eingabe-tomographischen Bild (22a) eingeschlossenes Pixel in Einheiten des Pixels ein spezifischer Läsionsbereich ist oder nicht, und einem zweiten maschinellen Lernmodell (2b), das klassifiziert, ob ein Bildblock, der durch Unterteilen des Eingabe-tomographischen Bildes (22a) in bestimmte Größen erhalten wird, in Einheiten des Bildblocks der spezifische Läsionsbereich ist oder nicht, auf der Grundlage von unter einer Vielzahl von tomographischen Bildern (22a), die durch Aufnahme eines Inneren eines oder mehrerer zweiter menschlicher Körper (11) erhalten werden, als Lerndaten verwendet werden, wenn das erste maschinelle Lernmodell (2a) durch maschinelles Lernen erzeugt wird, und von denen jedes den spezifischen Läsionsbereich einschließt, der Anzahl von tomographischen Bildern (22a), die an einer zweiten Bildaufnahmeposition des Inneren des einen oder der mehreren zweiten menschlichen Körper (11) aufgenommen werden, die der ersten Bildaufnahmeposition entspricht; und
Detektieren des spezifischen Läsionsbereichs aus dem ersten tomographischen Bild (22a) unter Verwendung von einem zwischen dem ersten maschinellen Lernmodell (2a) und dem zweiten maschinellen Lernmodell (2b) ausgewählten maschinellen Lernmodell;
wobei das zweite maschinelle Lernmodell (2b) den Läsionsbereich auf der Grundlage eines Merkmals in einem Unterteilungsbereich (A1-A3, B1-B3) anstelle des gesamten tomographischen Bildes (22a) klassifiziert.

## Revendications

1. Procédé de détection de lésion mis en œuvre par un ordinateur, le procédé comprenant :
l'acquisition d'une première image tomographique (22a) obtenue par imagerie d'une première position d'imagerie d'un intérieur d'un premier corps humain (22) ;
la sélection de l'un quelconque parmi un premier modèle d'apprentissage automatique (2a) qui classe si un pixel inclus dans une image tomographique d'entrée (22a) est ou non une région de lésion spécifique par unités du pixel et un deuxième modèle d'apprentissage automatique (2b) qui classe si un bloc d'images obtenu en divisant l'image tomographique d'entrée (22a) en certaines tailles est ou non la région de lésion spécifique en unités du bloc d'images, sur la base, parmi une pluralité d'images tomographiques (22a) qui sont obtenues par imagerie d'un intérieur d'un ou de plusieurs deuxièmes corps humains (11), sont utilisées comme données d'apprentissage lorsque le premier modèle d'apprentissage automatique (2a) est généré par apprentissage automatique et dont chacune inclut la région de lésion spécifique, du nombre d'images tomographiques (22a) capturées à une deuxième position d'imagerie de l'intérieur des un ou plusieurs deuxièmes corps humains (11), qui correspond à la première position d'imagerie ; et
la détection de la région de lésion spécifique à partir de la première image tomographique (22a) en utilisant un modèle d'apprentissage automatique sélectionné entre le premier modèle d'apprentissage automatique (2a) et le deuxième modèle d'apprentissage automatique (2b) ;
dans lequel le deuxième modèle d'apprentissage automatique (2b) classe la région de lésion sur la base d'une caractéristique dans une région de division (A1-A3, B1-B3) au lieu de l'image tomographique entière (22a).

2. Procédé de détection de lésion selon la revendication 1, comprenant en outre :
le comptage du nombre d'images tomographiques (22a) capturées dans une première région de division (A1-A3, B1-B3) correspondante parmi la pluralité d'images tomographiques (22a), pour chacune d'une pluralité de premières régions de division (A1-A3, B1-B3) obtenues en divisant une région d'organe prédéterminée dans les un ou plusieurs deuxièmes corps humains (11) en un nombre prédéterminé de divisions dans une direction prédéterminée,
dans lequel, lors de la sélection,
une troisième région de division (A1-A3, B1-B3) dans laquelle la première image tomographique (22a) est capturée est spécifiée, parmi une pluralité de deuxièmes régions de division (A1-A3, B1-B3) obtenues en divisant la région d'organe dans le premier corps humain (22) en un nombre prédéterminé de divisions dans la direction prédéterminée, et
l'un quelconque parmi le premier modèle d'apprentissage automatique et le deuxième modèle d'apprentissage automatique (2b) est sélectionné, sur la base du nombre d'images comptées pour une quatrième région de division (A1-A3, B1-B3) qui correspond à la troisième région de division (A1-A3, B1-B3) parmi la pluralité de premières régions de division (A1-A3, B1-B3).

3. Procédé de détection de lésion selon la revendication 2,
dans lequel, lors de la sélection, le premier modèle d'apprentissage automatique (2a) est sélectionné dans un cas dans lequel un rapport du nombre d'images comptées pour la quatrième région de division (A1-A3, B1-B3) à un nombre total de la pluralité d'images tomographiques (22a) est supérieur à une valeur seuil prédéterminée, et le deuxième modèle d'apprentissage automatique (2b) est sélectionné dans un cas dans lequel le rapport est inférieur ou égal à la valeur seuil prédéterminée.

4. Programme de détection de lésion amenant un ordinateur à exécuter un processus comprenant :
l'acquisition d'une première image tomographique obtenue par imagerie d'une première position d'imagerie d'un intérieur d'un premier corps humain (22) ;
la sélection de l'un quelconque parmi un premier modèle d'apprentissage automatique (2a) qui classe si un pixel inclus dans une image tomographique d'entrée (22a) est ou non une région de lésion spécifique par unités du pixel et un deuxième modèle d'apprentissage automatique (2b) qui classe si un bloc d'images obtenu en divisant l'image tomographique d'entrée (22a) en certaines tailles est ou non la région de lésion spécifique en unités du bloc d'images, sur la base, parmi une pluralité d'images tomographiques (22a) qui sont obtenues par imagerie d'un intérieur d'un ou de plusieurs deuxièmes corps humains (11), sont utilisées comme données d'apprentissage lorsque le premier modèle d'apprentissage automatique (2a) est généré par apprentissage automatique et dont chacune inclut la région de lésion spécifique, du nombre d'images tomographiques (22a) capturées à une deuxième position d'imagerie de l'intérieur des un ou plusieurs deuxièmes corps humains (11), qui correspond à la première position d'imagerie ; et
la détection de la région de lésion spécifique à partir de la première image tomographique (22a) en utilisant un modèle d'apprentissage automatique sélectionné entre le premier modèle d'apprentissage automatique (2a) et le deuxième modèle d'apprentissage automatique (2b) ;
dans lequel le deuxième modèle d'apprentissage automatique (2b) classe la région de lésion sur la base d'une caractéristique dans une région de division (A1-A3, B1-B3) au lieu de l'image tomographique entière (22a).
